(19) Europäisches Patentamt
European Patent Office
Office européen des brevets

(11) **EP 4 509 160 A1**

(12) **EUROPEAN PATENT APPLICATION**
published in accordance with Art. 153(4) EPC

(43) Date of publication:
**19.02.2025 Bulletin 2025/08**

(51) International Patent Classification (IPC):
***A61M 25/09*** *(2006.01)*

(21) Application number: **22937494.7**

(52) Cooperative Patent Classification (CPC):
**A61M 25/09**

(22) Date of filing: **15.04.2022**

(86) International application number:
**PCT/JP2022/017936**

(87) International publication number:
**WO 2023/199517 (19.10.2023 Gazette 2023/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: ASAHI INTECC CO., LTD.
**Seto-shi, Aichi, 489-0071 (JP)**

(72) Inventors:
• **NISHIO Kanako**
  **Seto-shi, Aichi 489-0071 (JP)**
• **YAGI Ayano**
  **Seto-shi, Aichi 489-0071 (JP)**
• **HIROTA Takuya**
  **Seto-shi, Aichi 489-0071 (JP)**

(74) Representative: **Winter, Brandl - Partnerschaft
mbB**
**Alois-Steinecker-Straße 22
85354 Freising (DE)**

(54) **GUIDE WIRE**

(57) This guide wire comprises a core shaft. The core shaft includes: a tapered portion of which an outer diameter increases toward a rear end side of the core shaft; and a straight portion having a substantially constant outer diameter in a longitudinal direction of the core shaft, which is provided on a rear end side of the tapered portion. When the outer diameter of a distal end of the tapered portion is represented by Da, the outer diameter of a rear end of the tapered portion is represented by Db, the longitudinal direction length of the tapered portion is represented by Lt (mm), and the longitudinal direction length of the straight portion is represented by Ls (mm), the following expressions (1) and (2) are satisfied.

$$0.1 \le (Db^4/Da^4)/Lt \le 1.3 \ ... \ (1)$$

$$0.3 \le Ls/Lt \le 4.5 \ ... \ (2)$$

Fig.1

EP 4 509 160 A1

## Description

### TECHNICAL FIELD

The present invention relates to a guide wire.

### BACKGROUND ART

Conventionally, guide wires are known as medical instruments that are percutaneously inserted into a blood vessel, and used to treat a constricted part that has formed inside the blood vessel. As such a guide wire, Patent Literature 1 describes a guide wire having a function of passing through a lesion.

### CITATION LIST

Patent Literature

Patent Literature 1: JP 2016-64068 A

### SUMMARY OF INVENTION

A conventional guide wire can, for example, significantly bend locally or bend spirally due to a resistance received from a constricted part when the guide wire passes through a lesion inside a blood vessel from the hand side toward the terminal end side, and there has been room for improvement in that a force exerted by a practitioner that pushes forward the guide wire is not efficiently transmitted. For this reason, by controlling the bent shape of the guide wire during use, and efficiently transmitting the force exerted by the practitioner that pushes forward the guide wire, it may be possible to improve the penetration of the guide wire through a lesion.

### TECHNICAL PROBLEM

The present invention has been made in order to solve the problem described above, and an object of the present invention is to provide a guide wire that, by controlling the bent shape of the guide wire during use, is capable of efficiently transmitting a force exerted by a practitioner that pushes forward the guide wire, and has superior penetration through a lesion.

### SOLUTION TO PROBLEM

The present invention has been made to solve at least part of the problem described above, and can be implemented as the following aspects.

(1) According to an aspect of the present invention, a guide wire is provided. The guide wire includes a core shaft, wherein the core shaft includes a tapered portion of which an outer diameter increases toward a rear end side of the core shaft, and a straight portion having a substantially constant outer diameter in a longitudinal direction of the core shaft, which is provided on a rear end side of the tapered portion, and when an outer diameter of a distal end of the tapered portion is represented by Da, an outer diameter of a rear end of the tapered portion is represented by Db, a longitudinal direction length of the tapered portion is represented by Lt (mm), and a longitudinal direction length of the straight portion is represented by Ls (mm), the following expressions (1) and (2) are satisfied.

$$0.1 \leq (Db^4/Da^4)/Lt \leq 1.3 \ ... \ (1)$$

$$0.3 \leq Ls/Lt \leq 4.5 \ ... \ (2)$$

According to such a configuration, as a result of the shape of the core shaft satisfying both expressions (1) and (2), the amount of change in the flexural rigidity in the longitudinal direction is controlled, and in addition, by adjusting the length ratio between the tapered portion and the straight portion, it is possible to prevent the core shaft from significantly bending locally, or bending spirally. As a result, by efficiently transmitting the force exerted by the practitioner that pushes forward the guide wire, the penetration can be improved.

(2) In the guide wire of the aspect above, the outer diameter Da of the distal end of the tapered portion, the outer diameter Db of the rear end of the tapered portion, the longitudinal direction length Lt (mm) of the tapered portion, and the longitudinal direction length Ls (mm) of the straight portion may satisfy the following expressions (3) and (4).

$$0.2 \leq (Db^4/Da^4)/Lt \leq 1.1 \ ... \ (3)$$

$$0.9 \leq Ls/Lt \leq 2.8 \ ... \ (4)$$

According to such a configuration, the force exerted by the practitioner that pushes forward the guide wire can be efficiently transmitted, and the penetration can be further improved.

(3) In the guide wire of the aspect above, the outer diameter Da of the distal end of the tapered portion, the outer diameter Db of the rear end of the tapered portion, the longitudinal direction length Lt (mm) of the tapered portion, and the longitudinal direction length Ls (mm) of the straight portion may satisfy the following expressions (5) and (6).

$$0.2 \leq (Db^4/Da^4)/Lt \leq 0.8 \ ... \ (5)$$

$$0.9 \leq Ls/Lt \leq 2.2 \ ... \ (6)$$

According to such a configuration, by suppressing rotation of the distal end of the guide wire that is not intended by the practitioner, it becomes easier to advance the distal end of the guide wire in a direction that is intended by the practitioner, further reducing the possibility of damaging the blood vessel.

Note that the present invention can be implemented in various modes, such as a guide wire, a manufacturing method of a guide wire, a manufacturing method of a catheter, an endoscope, and a dilator.

BRIEF DESCRIPTION OF DRAWINGS

Fig. 1 is an explanatory diagram illustrating an overall configuration of a guide wire according to a first embodiment.
Fig. 2 is an explanatory diagram illustrating a longitudinal cross-section of the guide wire according to the first embodiment.
Fig. 3 is an explanatory diagram illustrating a side view of a distal tip of the guide wire according to the first embodiment.
Fig. 4 is an explanatory diagram illustrating a front view of the distal tip of the guide wire according to the first embodiment. Fig. 5 is an explanatory diagram illustrating a distal end portion of a core shaft of the guide wire according to the first embodiment.
Fig. 6 is a diagram showing the results of a penetration test and a rotation test.
Fig. 7 is a diagram plotting each sample based on the values of expressions (1) and (2).
Fig. 8 is an explanatory diagram illustrating a bent shape of a guide wire.
Fig. 9 is an explanatory diagram illustrating the method of the penetration test.
Fig. 10 is an explanatory diagram illustrating a distal end portion of a core shaft of a guide wire according to a second embodiment.
Fig. 11 is an explanatory diagram illustrating a distal end portion of a core shaft of a guide wire according to a third embodiment.
Fig. 12 is an explanatory diagram illustrating a longitudinal cross-section of a guide wire according to a fourth embodiment.
Fig. 13 is an explanatory diagram illustrating a longitudinal cross-section of a guide wire according to a fifth embodiment.
Fig. 14 is an explanatory diagram illustrating a longitudinal cross-section of a guide wire according to a sixth embodiment.
Fig. 15 is an explanatory diagram illustrating a side view of a distal tip of the guide wire according to the sixth embodiment.
Fig. 16 is an explanatory diagram illustrating a front view of the distal tip of the guide wire according to the sixth embodiment.

Fig. 17 is an explanatory diagram illustrating a plan view of the distal tip of the guide wire according to the sixth embodiment.
Fig. 18 is an explanatory diagram illustrating a perspective view of the distal tip of the guide wire according to the sixth embodiment.
Fig. 19 is an explanatory diagram illustrating a side view of a distal tip of a guide wire according to a seventh embodiment.
Fig. 20 is an explanatory diagram illustrating a front view of the distal tip of the guide wire according to the seventh embodiment.
Fig. 21 is an explanatory diagram illustrating a modification.
Fig. 22 is an explanatory diagram illustrating a bent shape of a guide wire.
Fig. 23 is an explanatory diagram illustrating a bent shape of a guide wire.
Fig. 24 is an explanatory diagram illustrating a bent shape of a guide wire.

EMBODIMENTS OF THE INVENTION

A guide wire is a medical instrument that is inserted by a physician or the like into a blood vessel or digestive organ, and is used in treatment and examinations.

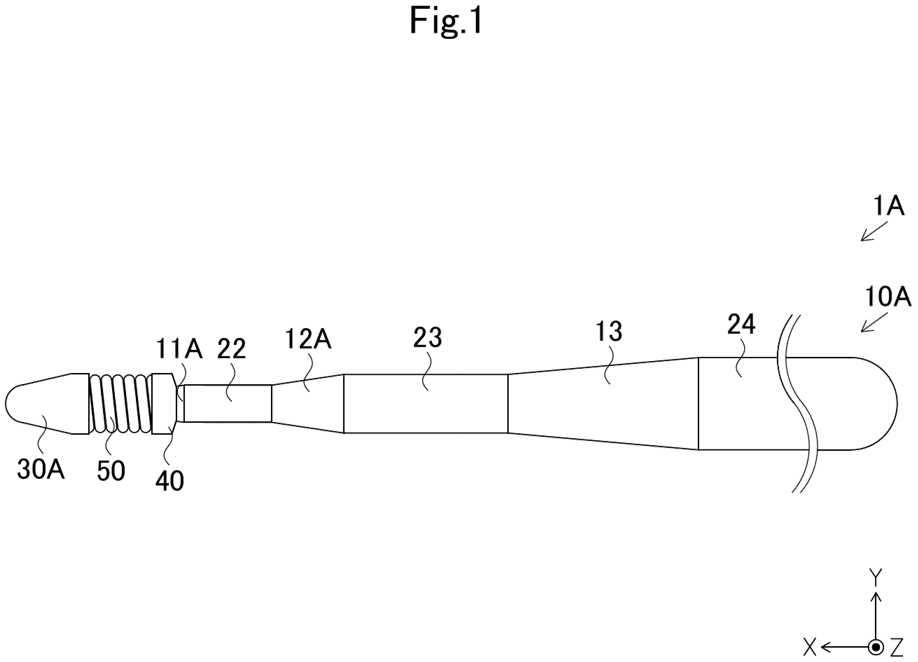

Hereinafter, the left side in each diagram that is illustrated from the Z axis direction, such as Fig. 1, is referred to as the "distal end side" of the guide wire of the present invention and each constituent member of the guide wire, and the right side is referred to as the "rear end side" of the guide wire and each constituent member. The distal end side of the guide wire is the side that is inserted into the body first when the guide wire is inserted into the body, and the rear end side of the guide wire is the side (near side) that is operated by a professional such as a physician. Furthermore, the end portion located on the distal end side of the guide wire and each constituent member of the guide wire is described as the "distal end", and a portion including the "distal end" that extends partway from the distal end toward the rear end side is described as the "distal end portion". Similarly, the end portion located on the rear end side of the guide wire and each constituent member of the guide wire is described as the "rear end", and a portion including the "rear end" that extends partway from the rear end toward the distal end side is described as the "rear end portion".

For convenience of the description, each diagram includes portions where the guide wire and each constituent member of the guide wire are shown with a size whose relative ratio is different from the actual relative ratio.

Each diagram includes coordinate axes for the purpose of describing the invention. The coordinate axes are used for the purpose of describing the present invention, and are not part of the present invention.

<First Embodiment>

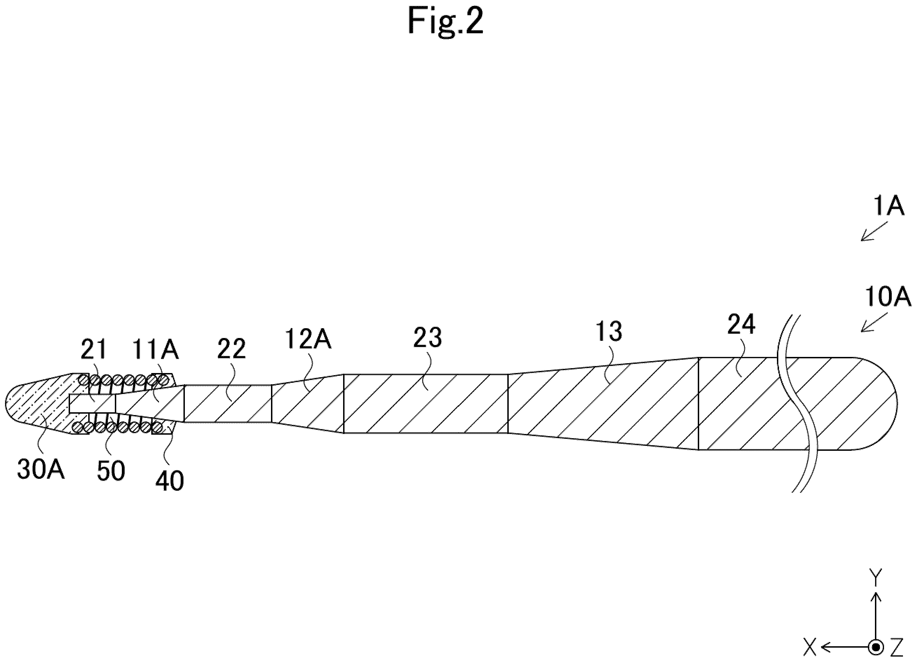

Fig. 1 is an explanatory diagram illustrating an overall configuration of a guide wire 1A according to a first embodiment. Fig. 2 is an explanatory diagram illustrating a longitudinal cross-section of the guide wire 1A according to the first embodiment.

The guide wire 1A is a medical instrument that is inserted by a physician or the like into a blood vessel or digestive organ, and is used in treatment and examinations. The guide wire 1A includes a core shaft 10A, and a coil 50 that covers a portion of the outer periphery of the core shaft 10A. A distal end portion of the core shaft 10A and a distal end portion of the coil 50 are fixed by a distal tip 30A. The core shaft 10A and a rear end portion of the coil 20 are fixed by a rear end side joint part 40.

The core shaft 10A has a total length of approximately 2,000 mm to 4,000 mm. The transverse cross-section of the core shaft 10A is circular, and the outer diameter is approximately 0.2 mm to 0.9 mm. The core shaft 10A is formed such that the outer diameter gradually becomes smaller from the rear end side toward the distal end side. The core shaft 10A includes straight portions (a first straight portion 21, a second straight portion 22, a third straight portion 23, and a fourth straight portion 24) and tapered portions (a first tapered portion 11A, a second tapered portion 12A, and a third tapered portion 13). The straight portions are parts of the core shaft 10A having a substantially constant outer diameter in the longitudinal direction. The tapered portions are parts of the core shaft 10A in which the outer diameter gradually becomes larger from the distal end side toward the rear end side. The core shaft 10A has the straight portions and the tapered portions alternately formed in the longitudinal direction. The core shaft 10A includes, from the distal end side toward the rear end side, the first straight portion 21, the first tapered portion 11A, the second straight portion 22, the second tapered portion 12A, the third straight portion 23, the third tapered portion 13, and the fourth straight portion 24. The first straight portion 21 is a part of the core shaft 10A having the smallest outer diameter. The fourth straight portion 24 is a part of the core shaft 10A having the largest outer diameter.

The core shaft 10A can be made of a material such as stainless alloy (such as SUS302, SUS304, or SUS316), piano wire, nickel-titanium alloy, nickel-chromium alloy, cobalt alloy, or tungsten. In the present embodiment, the core shaft 10A is made of SUS302.

The coil 50 is a member that is formed by winding a plurality of wires in a spiral shape to form a cylindrical shape. The core shaft 10A is disposed inside the coil 50. A gap is provided between the inner peripheral surface of the coil 50 and the outer peripheral surface of the core shaft 10A. The distal end portion of the coil 50 and the distal end portion of the core shaft 10A are fixed by the distal tip 30A. The coil 50 can be made of a material such as stainless alloy (such as SUS302, SUS304, or SUS316), piano wire, nickel-titanium alloy, nickel-chromium alloy, cobalt alloy, tungsten, or platinum.

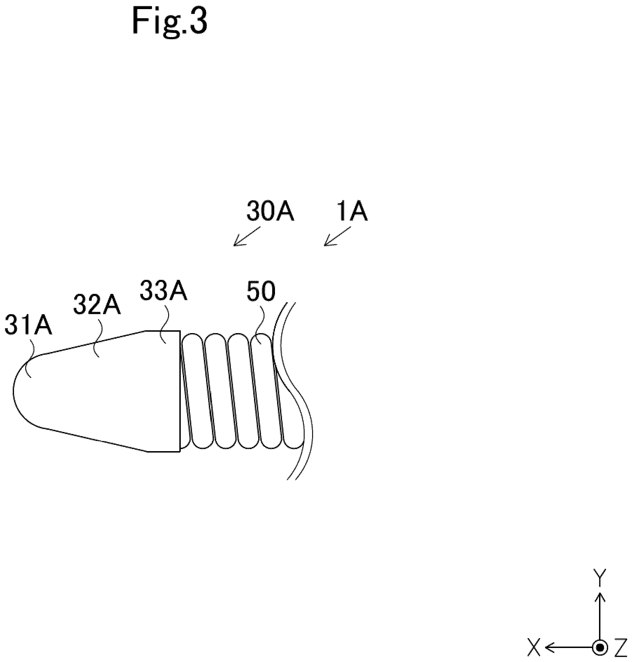

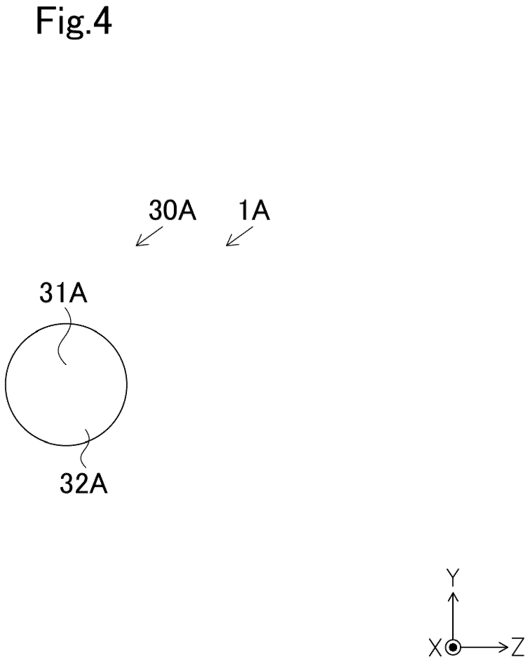

Figs. 3 and 4 are explanatory diagrams illustrating the guide wire 1A and the distal tip 30A according to the first embodiment. Figs. 3 and 4 are explanatory diagrams that respectively illustrate a side view and a front view of the distal tip 30A.

The distal tip 30A is a member that joins the distal end portion of the core shaft 10A and the distal end portion of the coil 50. The distal tip 30A is substantially circular in transverse cross-section. The distal tip 30A is formed such that the outer diameter gradually becomes smaller from the rear end side toward the distal end side. In other words, the distal tip has a tapered shape similar to an arrowhead. The distal tip 30A includes, from the distal end side toward the rear end side, a distal end portion 31A, an intermediate portion 32A, and a rear end portion 33A. The distal end portion 31A constitutes the most distal end of the distal tip 30A, and has a rounded shape. The intermediate portion 32A is located on the rear end side of the distal end portion 31A, and has a shape in which the outer diameter gradually becomes smaller toward the distal end side. The rear end portion 33A is located on the rear end side of the intermediate portion 32A, and the outer diameter is substantially constant in the longitudinal direction. The rear end portion 33A is connected to the distal end of the coil 50, and is a part that covers the outer periphery of the distal end of the coil 50. The distal tip 30A is formed using, for example, a metal solder (such as Au-Sn alloy, Sn-Ag alloy, Sn-Pb alloy, or Pb-Ag alloy), a brazing material (such as aluminum alloy solder, silver solder, or gold solder), or an adhesive (such as an epoxy-based adhesive).

The rear end side joint part 40 is a part that joins the core shaft 10A and the rear end portion of the coil 50. The rear end side joint part 40 is formed using, for example, a metal solder (such as Au-Sn alloy, Sn-Ag alloy, Sn-Pb alloy, or Pb-Ag alloy), a brazing material (such as aluminum alloy solder, silver solder, or gold solder), or an adhesive (such as an epoxy-based adhesive).

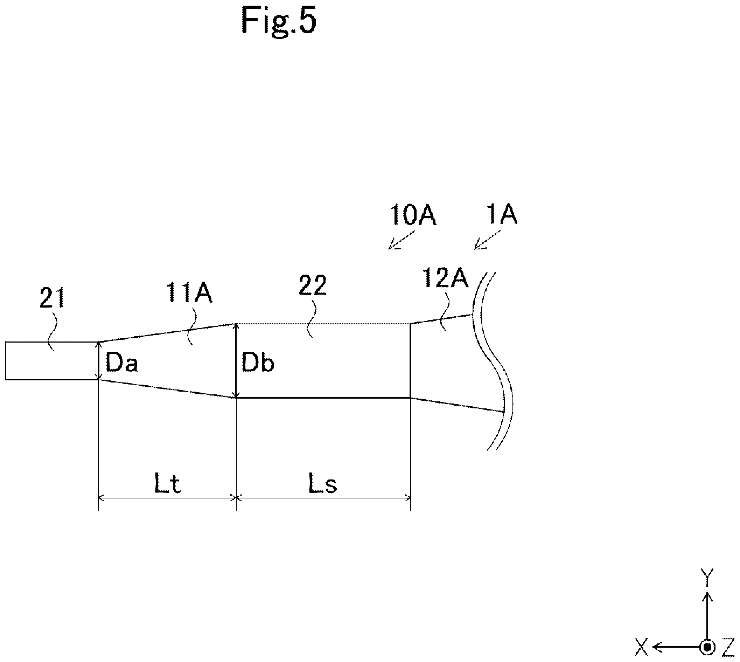

Fig. 5 is an explanatory diagram illustrating the distal end portion of the core shaft 10A of the guide wire 1A according to the first embodiment.

In the guide wire 1A, the outer diameter of the distal end of the first tapered portion 11A is represented by Da, and the outer diameter of the rear end of the first tapered portion 11A is represented by Db. The longitudinal direction length of the first tapered portion 11A is represented by Lt (mm), and the length of the second straight portion 22 is represented by Ls (mm).

The flexural rigidity of the distal end of the first tapered portion 11A is represented by FRa, the flexural rigidity of the rear end of the first tapered portion 11A is represented by FRb, and the ratio between them is represented by FRb/FRa. The first tapered portion 11A is made of SUS302, and has a substantially circular transverse cross-section. As a result, the flexural rigidity ratio FRb/FRa can be expressed as $Db^4/Da^4$. The value

$(Db^4/Da^4)/Lt$ obtained by dividing $Db^4/Da^4$ by the length Lt of the first tapered portion 11A is referred to as the rate of increase of the flexural rigidity in the longitudinal direction of the first tapered portion 11A. At this time, the rate of increase of the flexural rigidity in the longitudinal direction of the first tapered portion 11A satisfies expression (1) below.

$$0.1 \leq (Db^4/Da^4)/Lt \leq 1.3 \ ... \ (1)$$

The value Ls/Lt obtained by dividing the length Ls of the second straight portion 22 by the longitudinal direction length Lt of the first tapered portion 11A is referred to as the ratio of the length of the second straight portion 22 to the longitudinal direction length of the first tapered portion 11A. At this time, the ratio of the length of the second straight portion 22 to the longitudinal direction length of the first tapered portion 11A satisfies expression (2) below.

$$0.3 \leq Ls/Lt \leq 4.5 \ ... \ (2)$$

As a result of the outer diameter Da of the distal end of the first tapered portion 11A, the outer diameter Db of the rear end of the first tapered portion 11A, the longitudinal direction length Lt (mm) of the first tapered portion 11A, and the length Ls (mm) of the second straight portion 22 satisfying expressions (1) and (2) above, it is possible to prevent the core shaft 10A from significantly bending locally, or bending spirally. As a result, by efficiently transmitting the force exerted by the practitioner that pushes forward the guide wire, the penetration can be improved. The reason for this will be described using the results of a penetration test and a rotation test described below.

It is more preferable that the outer diameter Da of the distal end of the first tapered portion 11A, the outer diameter Db of the rear end of the first tapered portion 11A, the longitudinal direction length Lt (mm) of the first tapered portion 11A, and the length Ls (mm) of the second straight portion 22 satisfy expressions (3) and (4) below.

$$0.2 \leq (Db^4/Da^4)/Lt \leq 1.1 \ ... \ (3)$$

$$0.9 \leq Ls/Lt \leq 2.8 \ ... \ (4)$$

As a result of the outer diameter Da of the distal end of the first tapered portion 11A, the outer diameter Db of the rear end of the first tapered portion 11A, the longitudinal direction length Lt (mm) of the first tapered portion 11A, and the length Ls (mm) of the second straight portion 22 satisfying expressions (3) and (4) above, the force exerted by the practitioner that pushes forward the guide wire can be more efficiently transmitted, and the penetration can be further improved.

It is even more preferable that the outer diameter Da of the distal end of the first tapered portion 11A, the outer diameter Db of the rear end of the first tapered portion 11A, the longitudinal direction length Lt (mm) of the first tapered portion 11A, and the length Ls (mm) of the second straight portion 22 satisfy expressions (5) and (6) below.

$$0.2 \leq (Db^4/Da^4)/Lt \leq 0.8 \ ... \ (5)$$

$$0.9 \leq Ls/Lt \leq 2.2 \ ... \ (6)$$

As a result of the outer diameter Da of the distal end of the first tapered portion 11A, the outer diameter Db of the rear end of the first tapered portion 11A, the longitudinal direction length Lt (mm) of the first tapered portion 11A, and the length Ls (mm) of the second straight portion 22 satisfying expressions (5) and (6) above, rotation of the distal end of the guide wire that is not intended by the practitioner can be further suppressed. Consequently, it becomes even easier to advance the distal end of the guide wire in a direction that is intended by the practitioner, further reducing the possibility of damaging the blood vessel.

<Performance Evaluation Test>

As described below, in order to clarify the preferable numerical value ranges of the rate of increase $(Db^4/Da^4)/Lt$ of the flexural rigidity in the longitudinal direction of the first tapered portion 11A, and the ratio Ls/Lt of the length of the second straight portion 22 to the longitudinal direction length of the first tapered portion 11A, a penetration test and a rotation test were performed with respect to 19 samples having different values of $(Db^4/Da^4)/Lt$ and Ls/Lt.

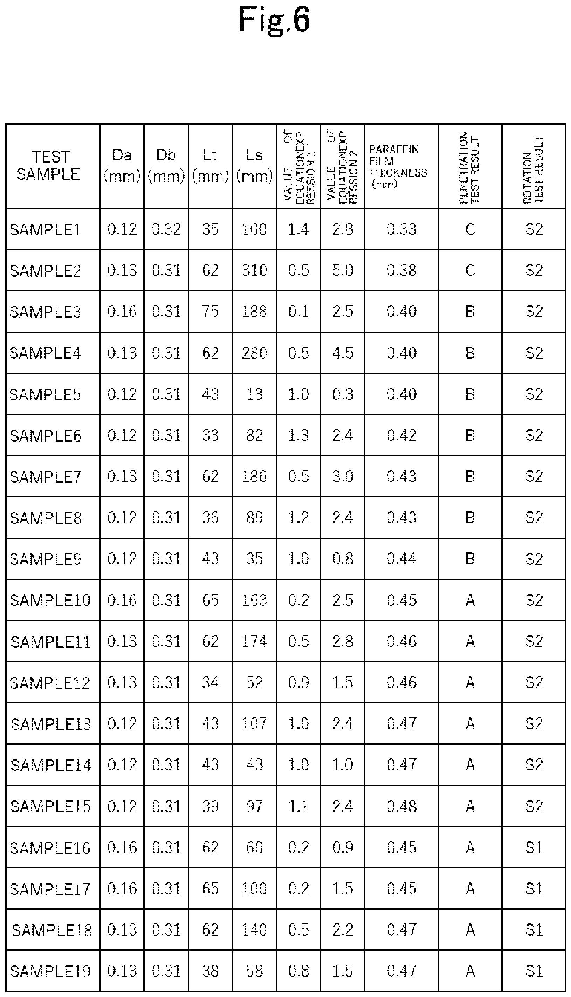

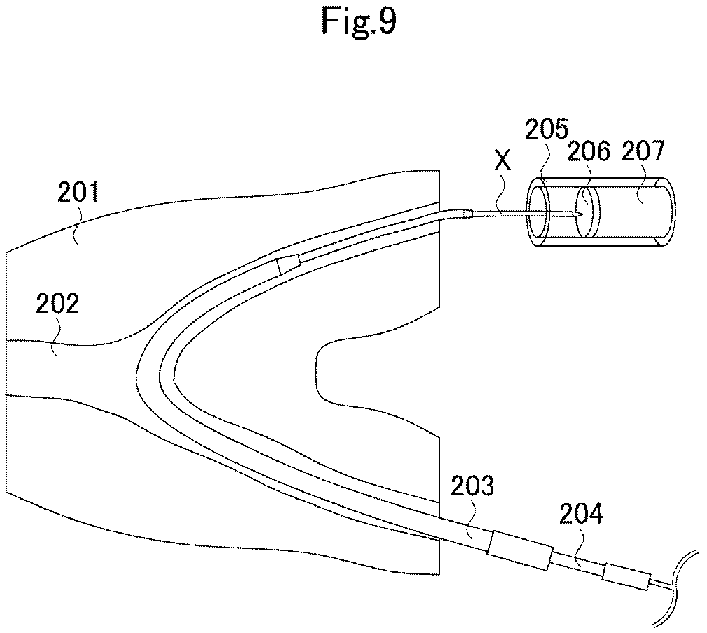

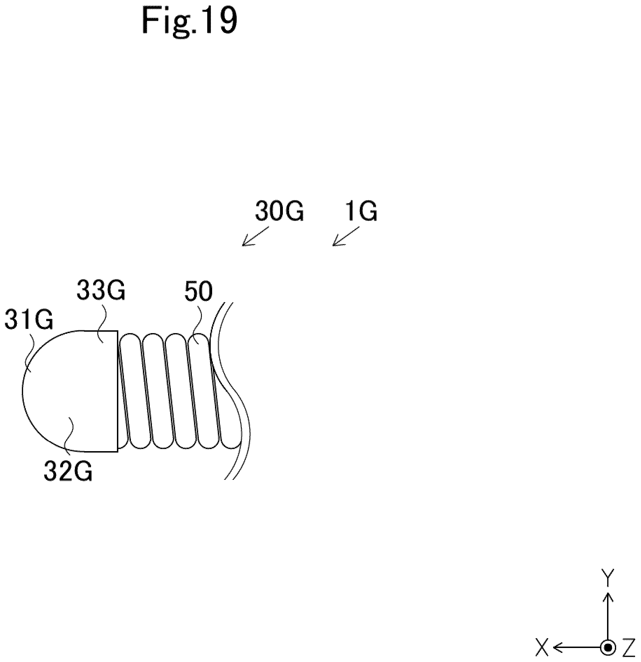

Fig. 6 is a diagram showing the results of the penetration test and the rotation test. As shown in Fig. 6, 19 types of samples (samples 1 to 19) were prepared, each having a different outer diameter Da (mm) of the distal end of the first tapered portion 11A, outer diameter Db (mm) of the rear end of the first tapered portion 11A, longitudinal direction length Lt (mm) of the first tapered portion 11A, and length Ls (mm) of the second straight portion 22. Using these samples, the penetration test was carried out using the method shown in Fig. 9 to confirm the effect of the values of $(Db^4/Da^4)/Lt$ and Ls/Lt on the bent shape of the core shaft 10A and the associated effect on the penetration.

<Penetration Test>

Fig. 9 is an explanatory diagram illustrating the method of the penetration test. In the penetration test, paraffin films with a thickness difference of 0.01 mm were prepared for samples 1 to 19, which each had different values of $(Db^4/Da^4)/Lt$ and Ls/Lt, and the penetration test was performed in order from the thinnest film thickness. The maximum thickness of the paraffin film that each sample was capable of penetrating through was used as an index for evaluating the penetration performance of the sample. The specific method of the penetration test will be described later.

<Rotation Test>

The rotation test was performed using the same test model as the penetration test in Fig. 9. The rotation angle of the distal end of the sample when pressed against a paraffin film was measured for each of samples 1 to 19, and the rotation angle was used as an evaluation index of the rotation performance of the sample.

<Test Results>

Under "penetration test result" in Fig. 6, a penetration test result of C was assigned to those samples in which the film thickness of the paraffin film that could be penetrated in the penetration test was less than 0.40 mm, a penetration test result of B was assigned to those samples in which the film thickness of the paraffin film that could be penetrated was 0.40 mm or more and less than 0.45 mm, and a penetration test result of A was assigned to those samples in which the film thickness of the paraffin film that could be penetrated was 0.45 mm or more. The penetration test result of samples 1 and 2 was C, the penetration test result of samples 3 to 9 was B, and the penetration test result of samples 10 to 19 was A.

Under "rotation test result" in Fig. 6, a rotation test result of S2 was assigned to those samples in which the rotation angle of the distal end in the rotation test was greater than 90 degrees, and a rotation test result of S1 was assigned to those samples in which the rotation angle of the distal end was 90 degrees or less. The rotation test result of samples 16 to 19 was S1, and the rotation test result of samples 1 to 15 was S2.

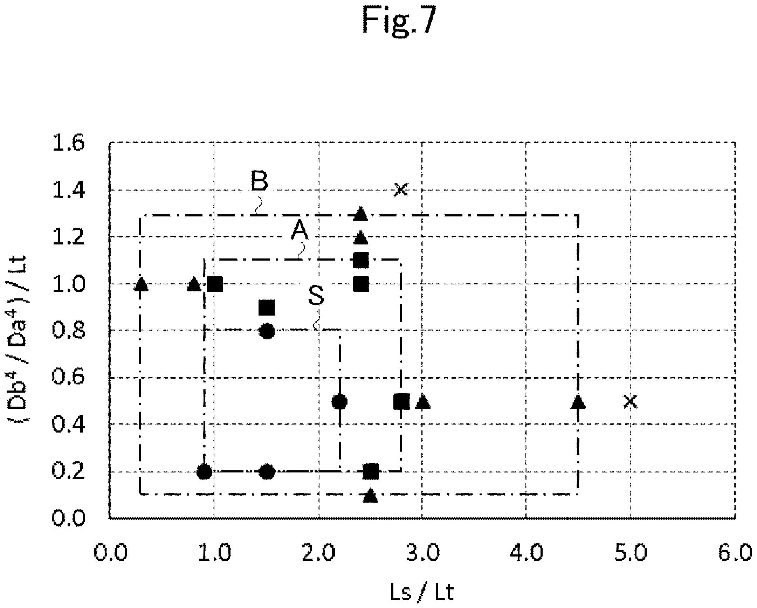

Fig. 7 is a diagram plotting each sample based on the values of $(Db^4/Da^4)/Lt$ and $Ls/Lt$. The vertical axis in Fig. 7 represents the value of $(Db^4/Da^4)/Lt$, and the horizontal axis represents the value of $Ls/Lt$. The plot shape of each sample 1 to 19 was assigned as follows according to the penetration test result and the rotation test result of each sample.

    Penetration test result: C; Rotation test result: S2 - X symbol
    Penetration test result: B; Rotation test result: S2 - triangle symbol
    Penetration test result: A; Rotation test result: S2 - square symbol
    Penetration test result: A; Rotation test result: S1 - circle symbol

The sample group having a penetration test result of B (samples 3 to 9) had a value of $(Db^4/Da^4)/Lt$ of 0.1 or more and 1.3 or less, and a value of $Ls/Lt$ of 0.3 or more and 4.5 or less. The numerical value ranges of $(Db^4/Da^4)/Lt$ and $Ls/Lt$ represented by the sample group having a penetration test result of B is displayed as a numerical value range B on the graph.

The sample group having a penetration test result of A (samples 10 to 19) had a value of $(Db^4/Da^4)/Lt$ of 0.2 or more and 1.1 or less, and a value of $Ls/Lt$ of 0.9 or more and 2.8 or less. The numerical value ranges of $(Db^4/Da^4)/Lt$ and $Ls/Lt$ represented by the sample group having a penetration test result of A is displayed as a numerical value range A on the graph. As shown in Fig. 7, the numerical value range A is a range that is included in the numerical value range B. Furthermore, the sample group having a penetration test result of C (samples 1 and 2) are not included in either the numerical value range A or the numerical value range B.

In comparison to the sample group that is not included in either the numerical value range A or the numerical value range B, the sample group included in the numerical value range B is capable of preventing the core shaft from significantly bending locally, or bending spirally. As a result, it was confirmed that, by efficiently transmitting the force exerted by the practitioner that pushes forward the guide wire, the penetration can be improved.

In comparison to the sample group that is included in the numerical value range B and the sample group that is not included in either the numerical value range A or the numerical value range B, the sample group included in the numerical value range A has an enhanced effect with respect to preventing the core shaft from significantly bending locally, or bending spirally. As a result, it was confirmed that, by more efficiently transmitting the force exerted by the practitioner that pushes forward the guide wire, the penetration can be improved even further.

For samples 1 to 19, a test was carried out in the same test system as the penetration test to determine whether or not a rotation that is not intended by the operator occurs at the distal end of the sample when the sample is pressed against a paraffin film. In the rotation test results in Fig. 6, the samples in which rotation occurred are represented by S2, and the samples in which rotation did not occur are represented by S1. The rotation test result of samples 1 to 15 was S2, and the rotation test result of samples 16 to 19 was S1.

In Fig. 7, those samples having a penetration test result of A and a rotation test result of S1 are plotted with circle symbols. The sample group having a rotation test result of S1 (samples 16 to 19) had a value of $(Db^4/Da^4)/Lt$ of 0.2 or more and 0.8 or less, and a value of $Ls/Lt$ of 0.9 or more and 2.2 or less. The numerical value ranges of $(Db^4/Da^4)/Lt$ and $Ls/Lt$ represented by the sample group having a rotation test result of S1 is displayed as a numerical value range S on the graph. The samples having a rotation test result of S2 (samples 1 to 15) are not included in the numerical value range S.

It was confirmed that, in comparison to the sample group that is not included in the numerical value range S, the sample group included in the numerical value range S was capable of suppressing rotation about the long axis in a state where the guide wire 1A is bent, which occurs when the guide wire 1A is pressed against a lesion. As a result of suppressing rotation of the distal end of the guide wire that is not intended by the practitioner, it becomes easier to advance the distal end of the guide wire in a direction that is intended by the practitioner, which reduces the possibility of damaging the blood vessel.

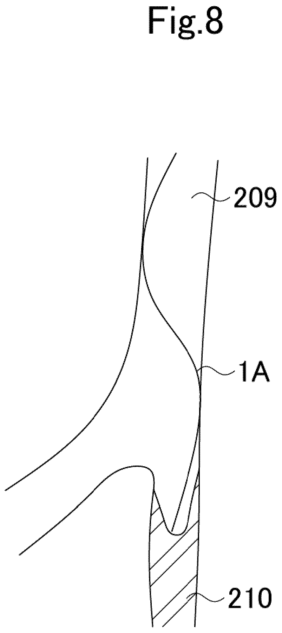

Fig. 8 is an explanatory diagram illustrating a bent shape of the guide wire 1A. Fig. 8 shows a state in which the guide wire 1A is inserted into a blood vessel of a human body, and the distal end of the guide wire 1A has reached a lesion 210 that has formed inside the blood vessel.

Fig. 8 shows a state in which the distal end of the guide wire 1A has reached the lesion 210 that has formed inside the blood vessel. The practitioner presses the distal end of the guide wire 1A against the surface of the lesion 210, and then attempts to push forward the guide wire 1A in order to penetrate the lesion. At this time, the guide wire 1 A becomes bent due to a resistance force received from the lesion 206. The guide wire 1A is evenly bent in the longitudinal direction. Here, evenly bent in the longitudinal direction refers to a state where, for example, there is no significant difference in the radius of curvature of each of the plurality of bends that repeat in the longitudinal direction, and each of the bends exists in approximately the same plane.

It was confirmed that the samples included in the numerical value range B, the numerical value range A, and the numerical value range S formed a bent shape that was evenly bent in the longitudinal direction. In other words, the guide wire 1A can be controlled to have a bent shape that is evenly bent in the longitudinal direction by having a value of $(Db^4/Da^4)/Lt$ of 0.1 or more and 1.3 or less, and a value of Ls/Lt of 0.3 or more and 4.5 or less. Furthermore, the guide wire 1A can be controlled to have a bent shape that is evenly bent in the longitudinal direction by having a value of $(Db^4/Da^4)/Lt$ of 0.2 or more and 1.1 or less, and a value of Ls/Lt of 0.9 or more and 2.8 or less. As a result, by efficiently transmitting the force exerted by the practitioner that pushes forward the guide wire, the penetration can be improved. In addition, as a result the guide wire 1A having a value of $(Db^4/Da^4)/Lt$ of 0.2 or more and 0.8 or less, and a value of Ls/Lt of 0.9 or more and 2.2 or less, which suppresses rotation of the distal end of the guide wire that is not intended by the practitioner, it becomes easier to advance the distal end of the guide wire in a direction that is intended by the practitioner, which reduces the possibility of damaging the blood vessel.

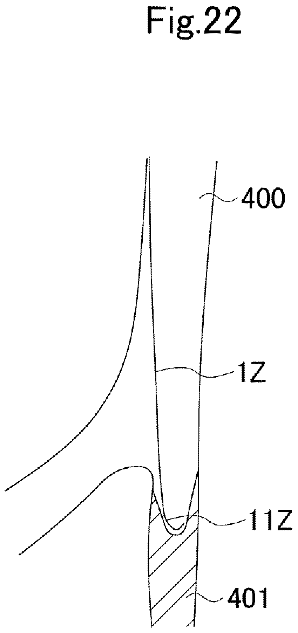

Figs. 22, 23, and 24 show a state in which the distal end of a guide wire 1Z that is not included in the embodiment of the present invention has reached a lesion 401 that has formed in a blood vessel 400.

Fig. 22 shows a state in which a first tapered portion 11Z is locally bent. The value of $(Db^4/Da^4)/Lt$ of the guide wire 1Z in Fig. 22 is greater than 1.3. When the value of $(Db^4/Da^4)/Lt$ of the guide wire 1Z is greater than 1.3, the rate of increase of the flexural rigidity in the longitudinal direction of the first tapered portion 11Z of the guide wire 1Z is large, and the possibility that local bending will occur at the first tapered portion 11Z increases. In such a case, it becomes difficult for the practitioner to efficiently transmit a force that pushes forward the guide wire 1Z.

On the other hand, when the value of $(Db^4/Da^4)/Lt$ of the guide wire 1Z is smaller than 0.1, the flexural rigidity of the guide wire 1Z becomes relatively small, and it becomes difficult for the practitioner to efficiently transmit a force that pushes forward the guide wire 1Z.

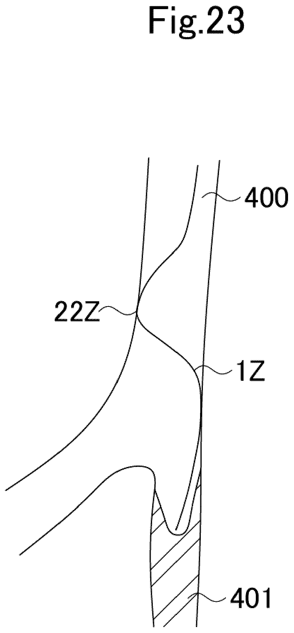

Fig. 23 shows a state in which a central portion of a straight portion 22Z is significantly bent. The value of Ls/Lt of the guide wire 1Z in Fig. 23 is greater than 4.5. When the value of Ls/Lt of the guide wire 1Z is greater than 4.5, the proportion of the total length of the guide wire 1Z accounted for by the second straight portion 22Z of the guide wire 1Z becomes large. As a result of the straight portion 22Z becoming significantly bent near the center, a force applied by the practitioner that pushes forward the guide wire tends to become dispersed in the bending direction. In such a case, it also becomes difficult for the practitioner to efficiently transmit a force that pushes forward the guide wire.

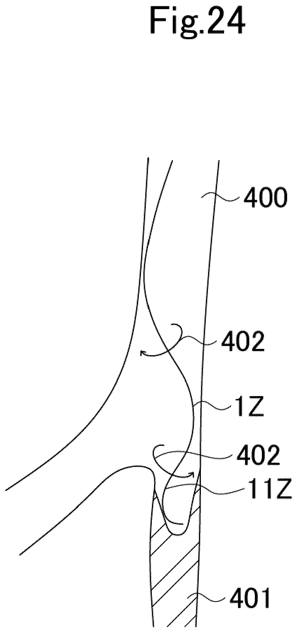

Fig. 24 shows a state in which the guide wire 1Z is bent in a spiral shape. The value of Ls/Lt of the guide wire 1Z in Fig. 24 is less than 0.3. When the value of Ls/Lt of the guide wire 1Z is smaller than 0.3, the proportion of the total length of the guide wire 1Z accounted for by the first tapered portion 11Z of the guide wire 1Z becomes large. When a significant bend occurs in the first tapered portion 11Z, the entire guide wire 1Z becomes bent in a spiral shape like a coil. When the practitioner operates the guide wire 1Z that has become bent in a spiral shape, a rotation that is not intended by the practitioner occurs. For example, a rotation is induced in the direction of a rotation 402. In such a case, it also becomes difficult for the practitioner to efficiently transmit a force that pushes forward the guide wire.

Fig. 9 is an explanatory diagram illustrating the method of the penetration test.

The penetration test was carried out according to the following method. First, a lower limb model 201 that simulates the lower limbs of a human body was installed. In the lower limb model 201, a simulated blood vessel 202 that simulates a blood vessel near the common iliac artery was formed. The lower limb model 201 was formed such that instruments could be inserted into the simulated blood vessel 202 through an opening of the simulated blood vessel 202 provided in a part that corresponds to the thigh. Because the opening of the simulated blood vessel 202 of one leg was connected to the opening of the simulated blood vessel 202 of the other leg, an instrument inserted through the opening of the simulated blood vessel 202 of one leg could be advanced to the opening of the simulated blood vessel 202 of the other leg. Next, a blood vessel model 205 was installed near the opening of the simulated blood vessel 202 of one leg. The blood vessel model 205 had a cylindrical shape that simulates a portion of a lower limb blood vessel of the human body, and therein was installed a lesion 207 that simulates a lesion that has formed inside the blood vessel, and a paraffin film 206 made of paraffin that was provided on the surface of the lesion 207. The hardness of the lesion was approximately 10,000 [gf/cm$^2$]. Next, a sheath 203 was inserted into the simulated blood vessel 202 from the opening of the simulated blood vessel 202 of the leg in which the blood vessel model 205 is not installed. The sheath 203 was indwelled in a state where the distal end of the sheath 203 had proceeded beyond a part corresponding to the common iliac artery. Then, a catheter 204 was inserted from the rear end of the sheath 203. The catheter 204 was advanced until the distal end of the catheter 204 was near the blood vessel model 205 and was then indwelled. Next, a sample X was inserted from the rear end of the catheter 204, advanced until the distal end of the sample X abuts against the surface of the paraffin film 206, and then indwelled. The sample X was a guide wire in which the values of expressions (1) and (2) had been distributed. Then, the sample X was pushed forward toward the paraffin film 206, and it was confirmed whether or not each sample could penetrate the paraffin film 206. A plurality of paraffin films 206 with thickness differences of 0.01 mm were prepared, and the tests were performed in order from the thinnest film thickness. The maximum thickness of the paraffin film 206 that each sample X was capable of penetrating through was used as an index for evaluating the penetration performance of the sample X.

The rotation test was carried out using the same test model as the penetration test. The description of the parts that are the same as the penetration test will be omitted. In the rotation test, a test was carried out to determine whether or not a rotation that is not intended by the operator occurred at the distal end of the sample X when the sample X was pressed against the paraffin film 206. The rotation angle of the distal end of the sample X was measured when the sample X was pressed against the paraffin film 206, and it was determined that a rotation had occurred when the rotation angle was greater than 90 degrees, and a rotation had not occurred when the rotation angle was 90 degrees or less. In the table of test results in Fig. 6, the samples in which rotation occurred are represented by S2, and the samples in which rotation did not occur are represented by S1.

<Second Embodiment>

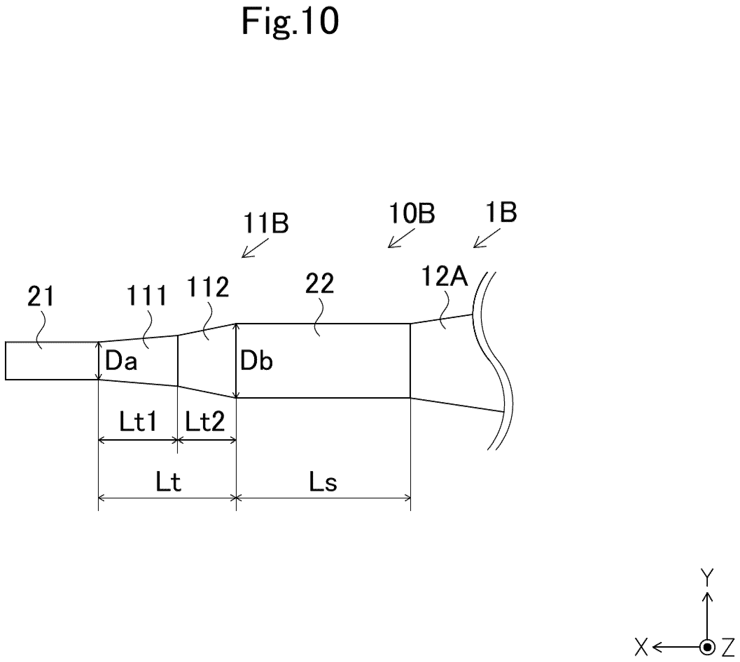

Fig. 10 is an explanatory diagram illustrating a distal end portion of a core shaft 10B of a guide wire 1B according to a second embodiment.

The guide wire 1B according to the second embodiment differs from the guide wire 1A according to the first embodiment in that it includes the core shaft 10B. The parts of the guide wire 1B other than the core shaft 10B are the same as those of the guide wire 1A, and the description thereof will be omitted.

The core shaft 10B includes a first tapered portion 11B. The first tapered portion 11B includes a distal end side tapered portion 111 and a rear end side tapered portion 112. The distal end side tapered portion 111 and the rear end side tapered portion 112 have a different degree of change (taper rate) in the flexural rigidity in the longitudinal direction. The amount of change in the flexural rigidity of the distal end side tapered portion 111 is smaller than the amount of change in the flexural rigidity of the rear end side tapered portion 112. When the length of the distal end side tapered portion 111 is represented by Lt1, and the length of the rear end side tapered portion 112 is represented by Lt2, the length Lt of the first tapered portion 11B is expressed as Lt1+Lt2. In the guide wire 1B according to the second embodiment, expressions (1) and (2) are expressed as follows. In expression (1), Da represents the outer diameter of the distal end of the first tapered portion 11B, and Db represents the outer diameter of the rear end of the first tapered portion 11B.

$$(Db^4/Da^4)/(Lt1+Lt2) \ldots (1)$$

$$Ls/(Lt1+Lt2) \ldots (2)$$

Even with a mode such as that of the guide wire 1B, the same effects as the guide wire 1A can be exhibited.

For example, a mode can be considered in which the amount of change of the flexural rigidity in the longitudinal direction of the first tapered portion is divided into three or more stages, and changes in a stepwise manner. Even with such a mode, the same effects as the guide wire 1A according to the first embodiment can be exhibited.

<Third Embodiment>

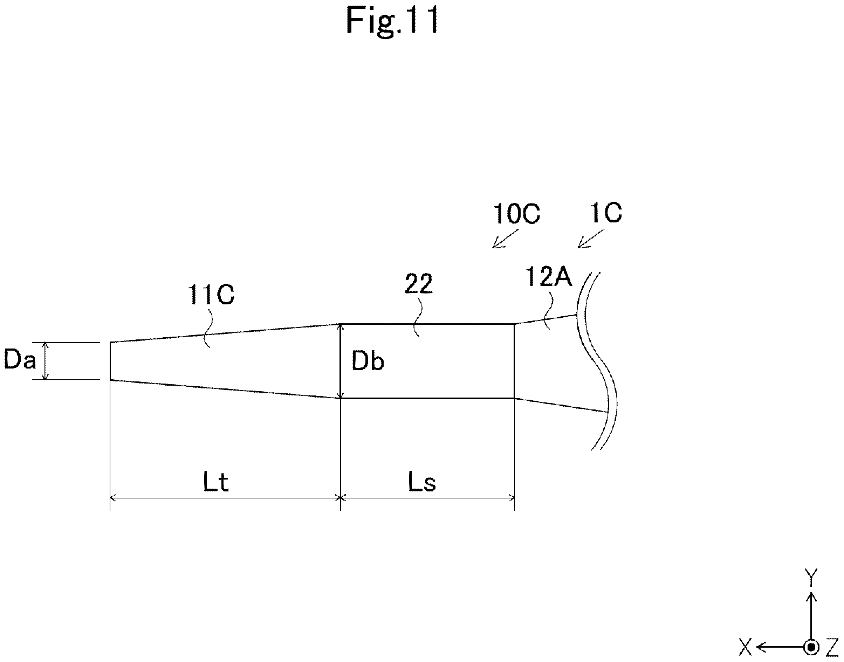

Fig. 11 is an explanatory diagram illustrating a distal end portion of a core shaft 10C of a guide wire 1C according to a third embodiment.

The guide wire 1C according to the third embodiment differs from the guide wire 1A according to the first embodiment in that it includes the core shaft 10C. The parts of the guide wire 1C other than the core shaft 10C are the same as those of the guide wire 1A, and the description thereof will be omitted.

The core shaft 10C does not include a part that corresponds to the first straight portion 21 of the guide wire 1A. The distal end of the core shaft 10C is formed of a first tapered portion 11C. A second straight portion 22 is provided on the rear end side of the first tapered portion 11C. In expression (1) of the guide wire 1C, Da represents the outer diameter of the distal end of the first tapered portion 11C, and Db represents the outer diameter of the rear end of the first tapered portion 11C. Even with a mode such as that of the guide wire 1C, the same effects as the guide wire 1A can be exhibited.

<Fourth Embodiment>

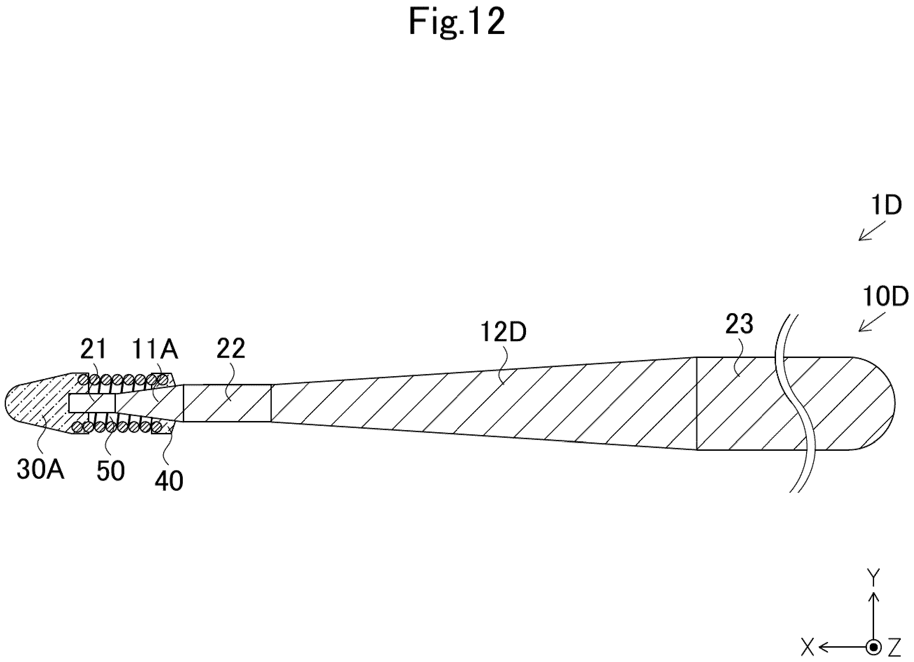

Fig. 12 is a diagram illustrating a guide wire 1D according to a fourth embodiment.

The guide wire 1D according to the fourth embodiment differs from the guide wire 1A according to the first embodiment in that it includes a core shaft 10D. The parts of the guide wire 1D according to the fourth embodiment other than the core shaft 10D are the same as those of the guide wire 1A according to the first embodiment, and the description thereof will be omitted.

The core shaft 10D includes, from the distal end side toward the rear end side, the first straight portion 21, the first tapered portion 11A, the second straight portion 22, a second tapered portion 12D, and the third straight portion 23. While the core shaft 10A has three tapered portions, the core shaft 10D has two tapered portions. Even with a mode such as that of the guide wire 1D, the same effects as the guide wire 1A according to the first embodiment can be exhibited.

<Fifth Embodiment>

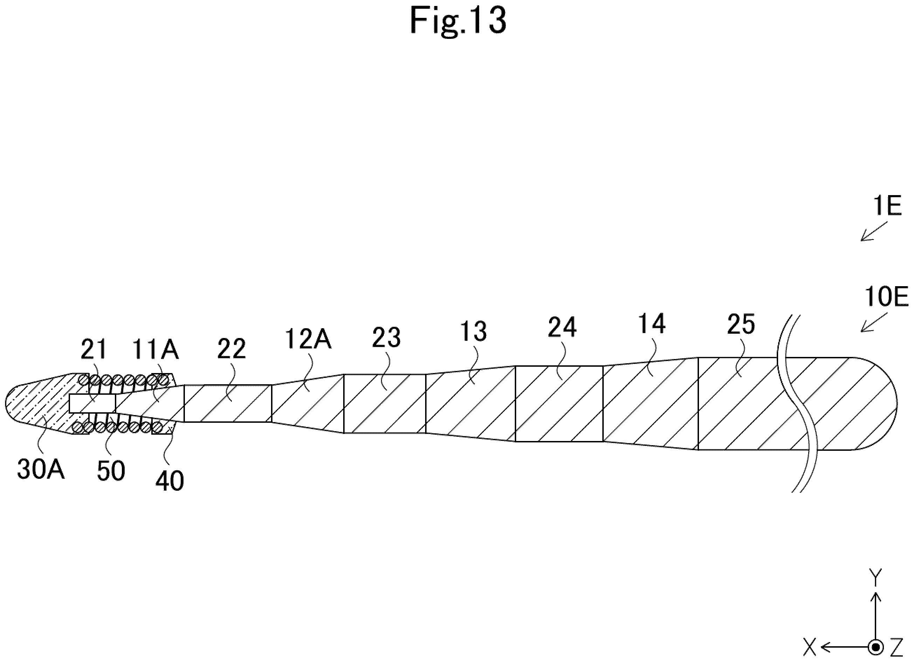

Fig. 13 is a diagram illustrating a guide wire 1E according to a fifth embodiment.

The guide wire 1E according to the fifth embodiment differs from the guide wire 1A according to the first embodiment in that it includes a core shaft 10E. The parts of the guide wire 1E other than the core shaft 10E are the same as those of the guide wire 1A, and the description thereof will be omitted.

The core shaft 10E includes, from the distal end side toward the rear end side, the first straight portion 21, the first tapered portion 11A, the second straight portion 22, the second tapered portion 12A, the third straight portion 23, the third tapered portion 13, the fourth straight portion 24, a fourth tapered portion 14, and a fifth straight portion 25. While the core shaft 10A has three tapered portions, the core shaft 10E has four tapered portions. Even with a mode such as that of the guide wire 1E, the same effects as the guide wire 1A according to the first embodiment can be exhibited.

<Sixth Embodiment>

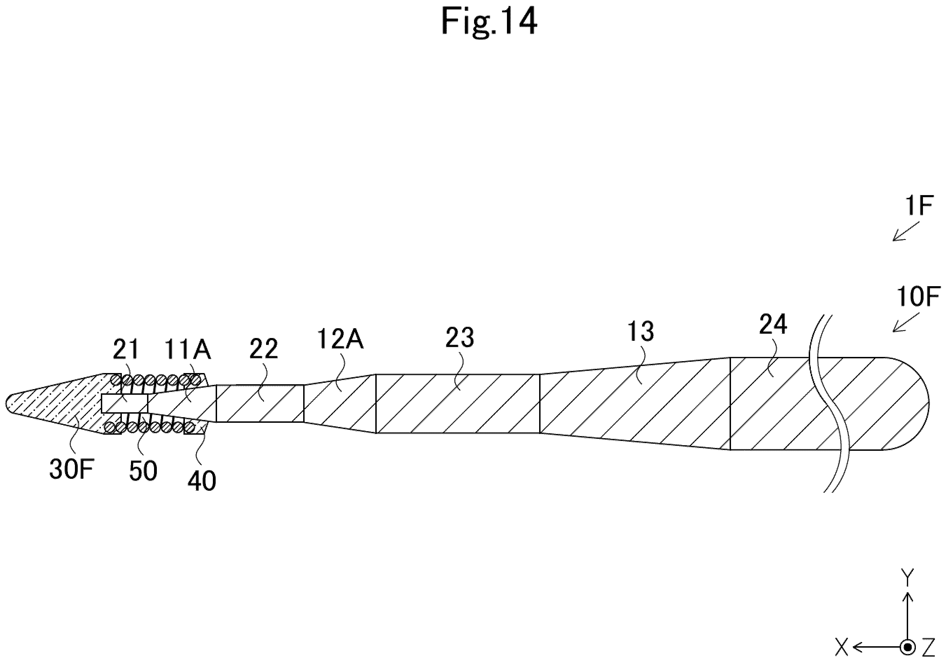

Fig. 14 is a diagram illustrating a guide wire 1F according to a sixth embodiment.

The guide wire 1F according to the sixth embodiment differs from the guide wire 1A according to the first embodiment in that it includes a distal tip 30F. The parts of the guide wire 1F other than the distal tip 30F are the same as those of the guide wire 1A, and the description thereof will be omitted.

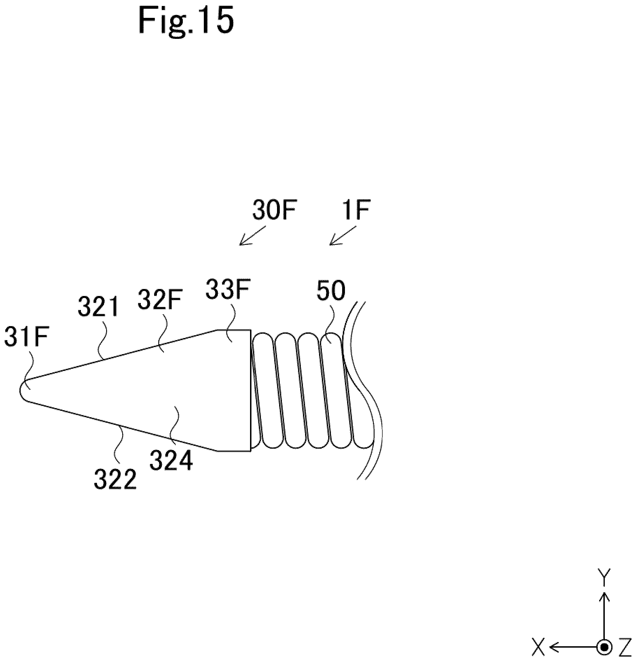

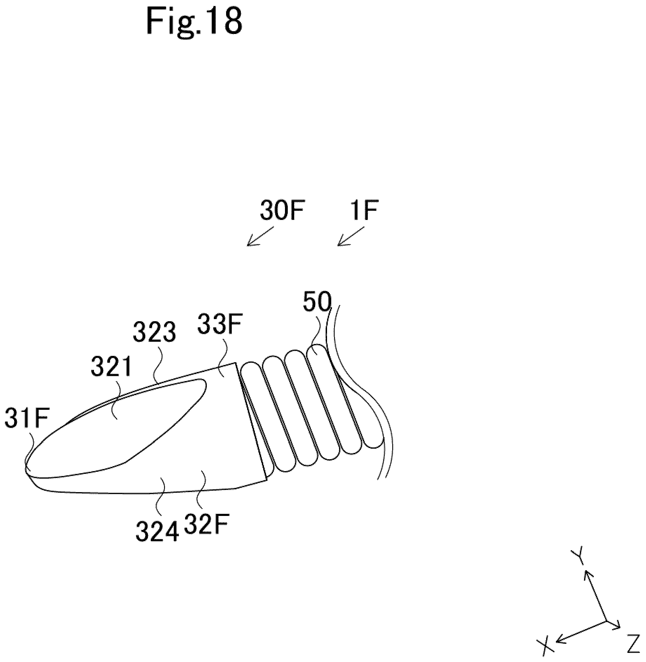

Figs. 15 and 18 are explanatory diagrams illustrating the distal tip 30F of the guide wire 1F according to a sixth embodiment. Figs. 15 to 18 are explanatory diagrams respectively illustrating a side view, a front view, a plan view, and a perspective view of the distal tip 30F.

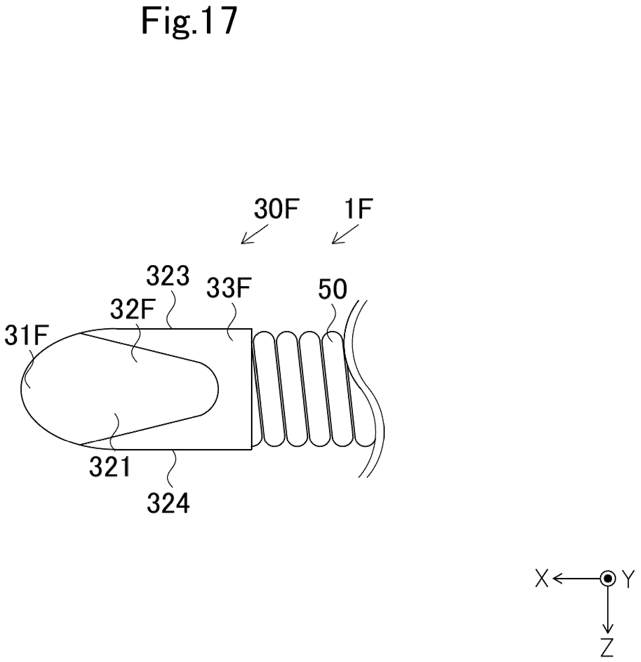

The distal tip 30F is formed such that the outer diameter gradually becomes smaller from the rear end side toward the distal end side. The distal tip 30F includes, from the distal end side toward the rear end side, a distal end portion 31F, an intermediate portion 32F, and a rear end portion 33F. The distal end portion 31F constitutes the most distal end of the distal tip 30F. As shown in Fig. 15, in a side view, the distal end portion 31F has a rounded shape. Furthermore, as shown in Fig. 17, in a plan view, the distal end portion 31F also has a rounded shape. The width (length in the Z axis direction) of the distal end portion 31F is formed so as to become smaller toward the distal end. In other words, the distal end portion 31F is formed having a convex shape toward the distal end.

The intermediate portion 32F is located on the rear end side of the distal end portion 31F, and has a shape in which the outer diameter gradually becomes smaller toward the distal end side. The intermediate portion 32F includes a first surface 321, a second surface 322, a third surface 323, and a fourth surface 324. The first surface 321 and the second surface 322 are formed so as to face each other. The first surface 321 and the second surface 322 have a nearly flat shape. As shown in Fig. 15, the first surface 321 and the second surface 322 are each inclined toward a central axis (not illustrated) of the distal tip 30F. The distal end portion 31F is formed between the distal end of the first surface 321 and the distal end of the second surface 322. The width (length in the Z axis direction) of the first surface 321 becomes larger toward the distal end. The rear end of the first surface 321 is formed having a convex shape (semicircular shape) toward the rear end. Similarly for the second surface 322, the width (length in the Z axis direction) of the second surface 322 becomes larger toward the distal end, and the rear end of the second surface 322 is formed having a convex shape (semicircular shape) toward the rear end.

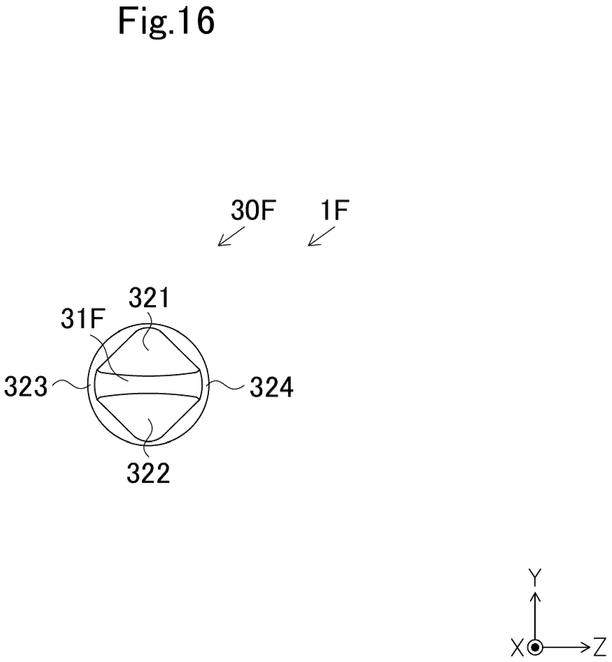

The third surface 323 and the fourth surface 324 are formed so as to face each other. The third surface 323 and the fourth surface 324 are provided between the first surface 321 and the second surface 322. The width (length in the Y axis direction) of the third surface 323 is formed so as to become smaller toward the distal end. The width (length in the Y axis direction) of the fourth surface 324 is similarly formed so as to become smaller toward the distal end. As shown in Fig. 16, in a front view, the third surface 323 and the fourth surface 324 are formed so as to project in opposite directions to each other. The third surface 323 has a rounded shape so as to project in the short axis direction of the distal tip 30F (negative Z axis direction). The fourth surface 324 has a rounded shape so as to project in the short axis direction of the distal tip 30F in the opposite direction to the third surface 323 (positive Z axis direction).

The rear end portion 33F is located on the rear end side of the intermediate portion 32F, and the outer diameter is substantially constant in the longitudinal direction. The rear end portion 33F is connected to the distal end of the coil 50, and is a part that covers the outer periphery of the distal end of the coil 50.

When the guide wire 1F reaches the surface of a lesion, the distal end portion 31F of the distal tip 30F can be hooked onto the surface of the lesion. As a result, the guide wire 1F can be more easily advanced into the lesion. Even with a mode such as that of the guide wire 1F, the same effects as the guide wire 1A according to the first embodiment can be exhibited.

<Seventh Embodiment>

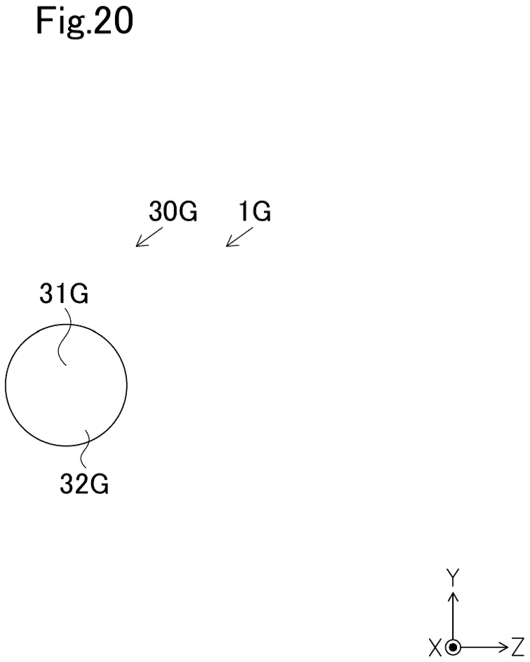

Figs. 19 and 20 are explanatory diagrams illustrating a distal tip 30G of a guide wire 1G according to a seventh embodiment. Figs. 19 and 20 are explanatory diagrams that respectively illustrate a side view and a front view of the distal tip 30G.

The guide wire 1G according to the seventh embodiment differs from the guide wire 1A according to the first embodiment in that it includes the distal tip 30G. The parts of the guide wire 1G other than the distal tip 30G are the same as those of the guide wire 1A, and the description thereof will be omitted.

The distal tip 30G has the distal end side formed having a hemispherical shape. The distal tip 30G is substantially circular in transverse cross-section. The distal tip 30G includes, from the distal end side toward the rear end side, a distal end portion 31G, an intermediate portion 32G, and a rear end portion 33G. The distal end portion 31G constitutes the most distal end of the distal tip 30G, and has a rounded shape. The intermediate portion 32G is located on the rear end side of the distal end portion 31G, and has a shape in which the outer diameter gradually becomes smaller toward the distal end side. The rear end portion 33G is located on the rear end side of the intermediate portion 32G, and the outer diameter is substantially constant in the longitudinal direction. The rear end portion 33G is connected to the distal end of the coil 50, and is a part that covers the outer periphery of the distal end of the coil 50. Even with a mode such as that of the guide wire 1G, the same effects as the guide wire 1A according to the first embodiment can be exhibited.

<Modification 1>

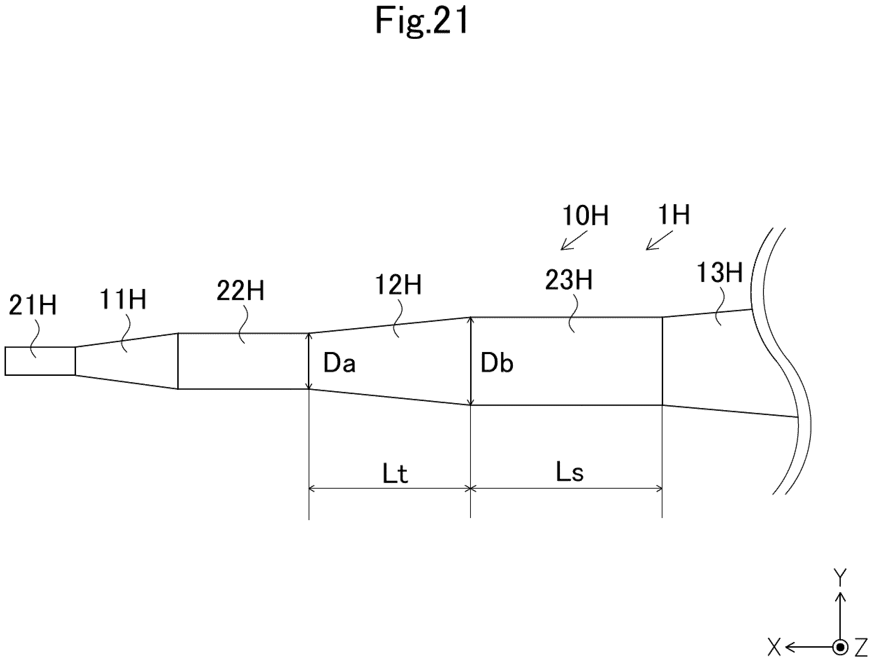

Fig. 21 is an explanatory diagram illustrating a modification of the guide wire 1A according to the first embodiment to the guide wire 1G according to the seventh embodiment.

Fig. 21 illustrates a guide wire 1H, which is a modification of the guide wire 1A according to the first embodiment to the guide wire 1G according to the seventh embodiment. In Fig. 21, the members other than the core shaft 10H are omitted. The core shaft 10H includes, from the distal end side toward the rear end side, a first straight portion 21H, a first tapered portion 11H, a second straight portion 22H, a second tapered portion 12H, a third straight portion 23H, and a third tapered portion 13H. The numerical value ranges of expressions (1) and (2) can be applied to the second tapered portion 12H and the third straight portion 23H. In this case, in the guide wire 1H, the outer diameter of the distal end of the second tapered portion 12H is represented by Da, and the outer diameter of the rear end of the second tapered portion 12H is represented by Db. Furthermore, the longitudinal direction length of the second tapered portion 12H can be represented by Lt, and the length of the third straight portion 23H can be represented by Ls. Even with a mode such as that of the guide wire 1H, the same effects as the guide wire 1A according to the first embodiment can be exhibited as a result of the values of expressions (1) and (2) being included in the numerical value range B, the numerical value range A, and the numerical value range S.

<Modification 2>

The guide wires according to the first embodiment to the seventh embodiment do not have a resin layer covering the outer periphery of the core shaft or the coil. However, a resin layer that covers the outer periphery of the core shaft or the coil may be included. For example, the outer periphery of the distal end side of the core shaft and the outer periphery of the coil may be covered with a hydrophilic resin layer, and the outer periphery of the rear end side of the core shaft may be covered with a hydrophobic resin layer. The hydrophilic resin layer may be, for example, polyvinylpyrrolidone, polyacrylic acid, polyacrylamide, polyvinyl alcohol, maleic anhydride copolymer, hyaluronic acid, and the like. The hydrophobic resin layer may be, for example, silicone oil, a fluororesin such as PTFE (polytetrafluoroethylene) or PFA (perfluoroalkoxyalkane), and the like.

<Modification 3>

The coil 50 of the guide wires according to the first embodiment to the seventh embodiment is a member formed by spirally winding a plurality of wires into a cylindrical shape, but the coil 50 may also be formed by spirally winding a single wire. Alternatively, the coil 50 may be formed by winding a strand, which is a single bundle of a plurality of wires, in a spiral shape so as to form a cylindrical shape.

DESCRIPTION OF REFERENCE NUMERALS

1A, 1B, 1C, 1D, 1E, 1F, 1G Guide wire
10A, 10B, 10C, 10D, 10E Core shaft
11A, 11B, 11C First tapered portion
12A, 12D Second tapered portion
13 Third tapered portion
14 Fourth tapered portion
21 First straight portion
22 Second straight portion
23 Third straight portion
24 Fourth straight portion
25 Fifth straight portion
30A, 30F, 30G Distal tip
31A, 31F, 31G Distal end portion
32A, 32F, 32G Intermediate portion
33A, 33F, 33G Rear end portion
40 Rear end side joint part
50 Coil
111 Distal end side tapered portion
112 Rear end side tapered portion
201 Lower limb model
202 Simulated blood vessel
203 Sheath
204 Catheter
205 Blood vessel model
206 Paraffin film
207 Simulated lesion
321 First surface
322 Second surface
323 First side surface
324 Second side surface
400 Blood vessel
401 Lesion
402 Rotation direction
A Numerical value range A
B Numerical value range B
S Numerical value range S
Da Outer diameter of distal end of tapered portion
Db Outer diameter of rear end of tapered portion
Ls Length of straight portion
Lt Length of tapered portion
Lt1 Length of distal end side tapered portion
Lt2 Length of rear end side tapered portion
X Sample

## Claims

1. A guide wire comprising a core shaft, wherein

   the core shaft includes
   a tapered portion of which an outer diameter increases toward a rear end side of the core shaft, and
   a straight portion having a substantially constant outer diameter in a longitudinal direction of the core shaft, which is provided on a rear end side of the tapered portion, and
when an outer diameter of a distal end of the tapered portion is represented by Da, an outer diameter of a rear end of the tapered portion is represented by Db, a longitudinal direction length of the tapered portion is represented by Lt (mm), and a longitudinal direction length of the straight portion is represented by Ls (mm), the following expressions (1) and (2) are satisfied.

$$0.1 \le (Db^4/Da^4)/Lt \le 1.3 \ ... \ (1)$$

$$0.3 \le Ls/Lt \le 4.5 \ ... \ (2)$$

2. The guide wire according to claim 1, wherein
   the outer diameter Da of the distal end of the tapered portion, the outer diameter Db of the rear end of the tapered portion, the longitudinal direction length Lt (mm) of the tapered portion, and the longitudinal direction length Ls (mm) of the straight portion satisfy the following expressions (3) and (4).

$$0.2 \le (Db^4/Da^4)/Lt \le 1.1 \ ... \ (3)$$

$$0.9 \le Ls/Lt \le 2.8 \ ... \ (4)$$

3. The guide wire according to claim 1, wherein
   the outer diameter Da of the distal end of the tapered portion, the outer diameter Db of the rear end of the tapered portion, the longitudinal direction length Lt (mm) of the tapered portion, and the longitudinal direction length Ls (mm) of the straight portion satisfy the following expressions (5) and (6).

$$0.2 \le (Db^4/Da^4)/Lt \le 0.8 \ ... \ (5)$$

$$0.9 \le Ls/Lt \le 2.2 \ ... \ (6)$$

# Fig.1

1A

10A

11A 22 12A 23 13 24

30A 50 40

EP 4 509 160 A1

12

Y

X ← ⊙ Z

# Fig.2

EP 4 509 160 A1

# Fig.3

EP 4 509 160 A1

Fig.4

30A    1A

31A

32A

EP 4 509 160 A1

Y

X → Z

# Fig.5

EP 4 509 160 A1

# Fig.6

| TEST SAMPLE | Da (mm) | Db (mm) | Lt (mm) | Ls (mm) | VALUE OF EQUATIONEXP RESSION 1 | VALUE OF EQUATIONEXP RESSION 2 | PARAFFIN FILM THICKNESS (mm) | PENETRATION TEST RESULT | ROTATION TEST RESULT |
|---|---|---|---|---|---|---|---|---|---|
| SAMPLE1 | 0.12 | 0.32 | 35 | 100 | 1.4 | 2.8 | 0.33 | C | S2 |
| SAMPLE2 | 0.13 | 0.31 | 62 | 310 | 0.5 | 5.0 | 0.38 | C | S2 |
| SAMPLE3 | 0.16 | 0.31 | 75 | 188 | 0.1 | 2.5 | 0.40 | B | S2 |
| SAMPLE4 | 0.13 | 0.31 | 62 | 280 | 0.5 | 4.5 | 0.40 | B | S2 |
| SAMPLE5 | 0.12 | 0.31 | 43 | 13 | 1.0 | 0.3 | 0.40 | B | S2 |
| SAMPLE6 | 0.12 | 0.31 | 33 | 82 | 1.3 | 2.4 | 0.42 | B | S2 |
| SAMPLE7 | 0.13 | 0.31 | 62 | 186 | 0.5 | 3.0 | 0.43 | B | S2 |
| SAMPLE8 | 0.12 | 0.31 | 36 | 89 | 1.2 | 2.4 | 0.43 | B | S2 |
| SAMPLE9 | 0.12 | 0.31 | 43 | 35 | 1.0 | 0.8 | 0.44 | B | S2 |
| SAMPLE10 | 0.16 | 0.31 | 65 | 163 | 0.2 | 2.5 | 0.45 | A | S2 |
| SAMPLE11 | 0.13 | 0.31 | 62 | 174 | 0.5 | 2.8 | 0.46 | A | S2 |
| SAMPLE12 | 0.13 | 0.31 | 34 | 52 | 0.9 | 1.5 | 0.46 | A | S2 |
| SAMPLE13 | 0.12 | 0.31 | 43 | 107 | 1.0 | 2.4 | 0.47 | A | S2 |
| SAMPLE14 | 0.12 | 0.31 | 43 | 43 | 1.0 | 1.0 | 0.47 | A | S2 |
| SAMPLE15 | 0.12 | 0.31 | 39 | 97 | 1.1 | 2.4 | 0.48 | A | S2 |
| SAMPLE16 | 0.16 | 0.31 | 62 | 60 | 0.2 | 0.9 | 0.45 | A | S1 |
| SAMPLE17 | 0.16 | 0.31 | 65 | 100 | 0.2 | 1.5 | 0.45 | A | S1 |
| SAMPLE18 | 0.13 | 0.31 | 62 | 140 | 0.5 | 2.2 | 0.47 | A | S1 |
| SAMPLE19 | 0.13 | 0.31 | 38 | 58 | 0.8 | 1.5 | 0.47 | A | S1 |

Fig.7

EP 4 509 160 A1

# Fig.8

209

1A

210

EP 4 509 160 A1

# Fig.9

Fig.10

# Fig.11

EP 4 509 160 A1

# Fig.12

EP 4 509 160 A1

# Fig.13

EP 4 509 160 A1

Fig.14

EP 4 509 160 A1

EP 4 509 160 A1

Fig.15

# Fig.16

EP 4 509 160 A1

Fig.17

EP 4 509 160 A1

Fig.18

# Fig.19

EP 4 509 160 A1

# Fig.20

EP 4 509 160 A1

30G  1G

31G

32G

Y

X⊙→Z

Fig.21

EP 4 509 160 A1

Fig.22

Fig.23

# Fig.24

400
402
1Z
402
11Z
401

**INTERNATIONAL SEARCH REPORT**

| International application No. |
| --- |
| **PCT/JP2022/017936** |

| A. | CLASSIFICATION OF SUBJECT MATTER |
| --- | --- |

*A61M 25/09*(2006.01)i
FI:  A61M25/09 550

According to International Patent Classification (IPC) or to both national classification and IPC

| B. | FIELDS SEARCHED |
| --- | --- |

Minimum documentation searched (classification system followed by classification symbols)

A61M25/09

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2011-120739 A (ASAHI INTECC CO LTD) 23 June 2011 (2011-06-23) paragraphs [0022]-[0026], fig. 1, 3 | 1-3 |
| X | US 2013/0110000 A1 (TERUMO MEDICAL CORPORATION) 02 May 2013 (2013-05-02) paragraph [0033], fig. 1 | 1-3 |
| A | JP 2002-522175 A (BOSTON SCIENTIFIC LIMITED) 23 July 2002 (2002-07-23) paragraph [0012], fig. 1 | 1-3 |
| A | JP 2006-519058 A (BOSTON SCIENTIFIC LIMITED) 24 August 2006 (2006-08-24) paragraphs [0029], [0046], fig. 3, 5 | 1-3 |
| A | WO 2019/073571 A1 (ASAHI INTECC CO LTD) 18 April 2019 (2019-04-18) entire text, all drawings | 1-3 |
| A | WO 2018/181177 A1 (TERUMO CORP) 04 October 2018 (2018-10-04) entire text, all drawings | 1-3 |
| A | US 5865767 A (CORDIS CORPORATION) 02 February 1999 (1999-02-02) entire text, all drawings | 1-3 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **12 May 2022** | **14 June 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

| | | International application No. |
|---|---|---|
| | | **PCT/JP2022/017936** |

| Patent document cited in search report | | | Publication date (day/month/year) | Patent family member(s) | | | Publication date (day/month/year) |
|---|---|---|---|---|---|---|---|
| JP | 2011-120739 | A | 23 June 2011 | US 2011/0144538 A1 paragraphs [0043]-[0056], fig. 1, 3 EP 2332608 A2 CN 102091380 A | | | |
| US | 2013/0110000 | A1 | 02 May 2013 | (Family: none) | | | |
| JP | 2002-522175 | A | 23 July 2002 | US 6106488 A column 3, lines 7-17, fig. 1 WO 2000/009191 A1 EP 1666083 A1 | | | |
| JP | 2006-519058 | A | 24 August 2006 | US 2004/0167440 A1 paragraphs [0041], [0059], fig. 3, 5 WO 2004/075964 A2 | | | |
| WO | 2019/073571 | A1 | 18 April 2019 | US 2020/0238054 A1 entire text, all drawings EP 3695874 A1 KR 10-2020-0054275 A CN 111225708 A | | | |
| WO | 2018/181177 | A1 | 04 October 2018 | US 2019/0329006 A1 entire text, all drawings EP 3603728 A1 CN 110198755 A | | | |
| US | 5865767 | A | 02 February 1999 | EP 818215 A1 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016064068 A